(19) Europäisches Patentamt
European Patent Office
Office européen des brevets

(11) Publication number: 0 185 350
A2

(12) # EUROPEAN PATENT APPLICATION

(21) Application number: 85116070.5

(22) Date of filing: 17.12.85

(51) Int. Cl.$^4$: C 07 C 102/00
C 07 C 103/133, C 07 C 103/58

(30) Priority: 19.12.84 US 683840

(43) Date of publication of application:
25.06.86 Bulletin 86/26

(84) Designated Contracting States:
BE DE FR GB IT NL

(71) Applicant: The B.F. GOODRICH Company
Dept. 0015 WHB-6 500 South Main Street
Akron, Ohio 44318(US)

(72) Inventor: Nicholas, Paul Peter
4775 Canterbury Lane
Broadview Heights Ohio 44147(US)

(74) Representative: von Kreisler, Alek, Dipl.-Chem. et al,
Deichmannhaus am Hauptbahnhof
D-5000 Köln 1(DE)

(54) **Process for making acrylamide and N-substituted acrylamides.**

(57) A process for making acrylamides and N-substituted acrylamides wherein vinyl chloride monomer, carbon monoxide, an amine, and a platinum group metal based catalyst complex are combined, and heated to at least 60°C under generally autogenous pressure.

# PROCESS FOR MAKING ACRYLAMIDE AND N-SUBSTITUTED ACRYLAMIDES

## FIELD OF THE INVENTION

This invention relates to acrylamide and N-substituted acrylamides and particularly to methods for making these acrylamides.

## BACKGROUND OF THE INVENTION

Acrylamides are a family of crystalline, easily polymerized monomers used in the synthesis of dyes, in adhesives, in paper and textile sizes, in soil conditioning agents, in treating sewage and waste water, in permanent press fabrics, enhancing oil recovery, and in the manufacture of flocculents.

Among successful initial processes, acrylamide was produced commercially by reacting approximately equal molar quantities of acrylonitrile, water and sulfuric acid together to form acrylamide sulfate. Acrylamide then was recovered from the sulfate salt, usually by ammonia "neutralization", removing the resulting insoluble ammonium sulfate by filtration, and crystallizing the acrylamide from the filtrate. Another procedure for recovering acrylamide from acrylamide sulfate involves the use of an ion-exchange technique in which acrylamide sulfate is diluted with water and passed through a cation-exchange resin (such as a sulfonated copolymer of styrene and divinylbenzene). The product is a water solution of acrylamide. The aforementioned sulfuric acid-catalyzed process for forming acrylamide has a number of drawbacks that made it desirable to find alternative methods for producing acrylamide.

In one improved commercial proposal, acrylamide is formed by the direct hydration of acrylonitrile using a catalyst that typically consists of a mixture of oxides of copper and chromium. While overcoming some difficulties associated with sulfuric acid, this process poses other difficulties in part related to a requirement for substantial dilution as well as the necessity for employing relatively expensive acrylonitrile as a starting material. Nevertheless, this process has found considerable commercial success. This process does have the advantage of producing no "waste products" of reaction. Acrylamide formed by this process typically is sold commercially as a 50 percent aqueous solution.

It previously has been reported that certain organobromides and organoiodides can form amides when reacted with carbon monoxide and organic amines using $PdCl_2(PPh_3)_2$ (Ph being phenyl) as a catalyst, A. Schoenberg and R. F. Heck, 39 Journal of Organic Chemistry, p. 3327 (1974), the disclosure therein being incorporated herein by reference. It also is reported that lactams can be prepared from bromoalkenes and iodoalkenes that have secondary amine substituents, M. Mori, Y. Washioka, T. Urayama, K. Chiba and Y. Ban, 48 Journal of Organic Chemistry, p. 4058 (1983), the disclosure therein being incorporated herein by reference and that benzolactams can be prepared from ortho-bromoaminoalkylbenzenes by photo-chemical carbonylation under phase transfer conditions using cobalt carbonyl as the catalyst, J. Brunet, C. Sidot and P. Caubere, 48 Journal of Organic Chemistry, p. 1166, (1983), the disclosure therein being incorporated herein by reference.

Although it has been reported that 2-chloropropene can be converted to an anilide by reaction with carbon monoxide, aniline, and a stoichiometric quantity of tri-n-butylamine, Schoenberg and Heck, supra, the reaction is extremely slow even at elevated temperatures and pressures, with an estimated turnover number (mol converted · mol catalyst$^{-1}$ · hour$^{-1}$) of between about 1-2 at 135°C and 4137 kPa (gauge). The very slow reaction rate would be expected in view of the low activity reported for alkenyl chlorides and aryl chlorides in metallation reactions compared with reaction rates of alkenyl and aryl bromides and iodides, P. Fitton and E. A. Rick, 28 Journal of Organometallic Chemistry, p. 287, (1971); J. T. Colman and L. S. Hegedus, Principles and Applications of Organotransition Metal Chemistry, p.p. 185 (1980); R. F. Heck and B. Patel, Catalysis in Organic Synthesis, Seventh Edition, p. 195-218; J. Brunet, C. Sidot and P. Caubere, 48 Journal of Organic Chemistry, p. 1166 (1983).

Even though organobromides and organoiodides have been reported to form amides when reacted with carbon monoxide and organic amines using $PdCl_2(PPh_3)_2$ as a catalyst, it has been found that vinyl chloride monomer does not react with carbon monoxide and ammonia when $PdCl_2(PPh_3)_2$ is used as a catalyst.

## SUMMARY OF THE INVENTION

The present invention pertains to the catalyzed reaction of (1) vinyl chloride monomer, (2) carbon monoxide, and (3) an amidation agent or amine, as hereinafter termed, to form acrylamides, the reaction being carried out desirably at moderately elevated temperature preferably of 90°C

or greater, and a pressure preferably at least autogenous. The reaction can be represented by the following chemical equation:

$$CH_2{=}CHCl + CO + 2HNR^{I}R^{II} \xrightarrow{\text{(catalyst)}}$$

$$CH_2{=}CH\overset{O}{\overset{\|}{C}}NR^{I}R^{II} + Cl^{-}H_2\overset{+}{N}R^{I}R^{II}$$

The term amine is intended to encompass primary and secondary amines and ammonia; the term acrylamides is intended to encompass acrylamide and N-substituted acrylamide. $R^{I}$, $R^{II}$ typically are alkyls, aromatics, or cycloalkyls having 1-10 carbon atoms.

The vinyl chloride monomer, carbon monoxide, and the amine are combined, the combination including a catalyst of the form $Y_zM(L)_n$ wherein L is one of $PR_3$ and $[R_2P\text{-}A\text{-}PR_2]$. The combination is maintained under agitation at a temperature at least in excess of about 60°C under pressure in order to initiate and sustain the reaction. Following completion of the reaction, acrylamide is recovered from the combination. Preferably the amine and carbon monoxide are replenished within the combination of reactants to preclude their exhaustion. In preferred homogenous or single phase reactions, typically liquid phase, vinyl chloride also functions as a solvent or liquid phase carrier. Where due to temperature, volume and pressure, about 30% to about 40% by weight of vinyl chloride charged to a reactor assumes a gaseous state, carbon monoxide is present in the combination at a partial pressure of at least 275 kPa and preferably

345 kPa (as measured at 21°C); preferably the amine is present in the combination in a mole ratio to the vinyl chloride of not less than 0.02; and preferably the catalyst is present in the combination in a quantity of not less than about 0.1 millimole per mole of vinyl chloride present in the liquid phase where a homogeneous phase reaction occurs in a liquid phase.

The catalyst is preferably a complex of a platinum group metal and phosphine and typically possesses the form $Y_zM(L)_n$ wherein L is one of $PR_3$ and $[R_2P\text{-}A\text{-}PR_2]$ wherein M is a platinum group metal, R is aryl, substituted aryl, or alkyl such as branched or straight chained alkyl, cyclohexyl, teriary-butyl and derivatives thereof having a substitutent X wherein methyl, low molecular weight halogen, fluoromethyl or hydrogen; R is typically phenyl, alkyl, or cyclohexyl and n=1-4 (2-4 where $L=PR_3$, and 1-2 where $L=[R_2P\text{-}A\text{-}PR_2]$), A is a connecting organic radical, M desirably is a palladium (O) or so-called zero valent palladium, complexed with a tertiary phosphine and z=o.

Typically Y is a monovalent anion, but may be divalent, typically being an integer, 0, 1, 2.

Vinyl chloride is unique among monochloroalkenes in having a catalyzed rate of reaction with carbon monoxide and ammonia unexpectedly orders of magnitude greater than a rate for its alkylated derivatives.

The above and other features and advantages of the invention will become apparent from the following detailed description of the invention which forms a part of the specification.

## BRIEF DESCRIPTION OF THE DRAWINGS

Fig. 1 is a schematic representation of a chemical reactor device in which the instant invention may be practiced.

## BEST EMBODIMENT OF THE INVENTION

The present invention provides a process for producing acrylamides, that is acrylamide and N-substituted acrylamides . In the process, reactants are combined with a catalyst and heated to a temperature, generally in excess of 60°C, and more typically in excess of 80°C, sufficient to activate and sustain the reaction among the reactants. Typically the reaction occurs at an elevated pressure; generally the pressure is autogenous pressure.

The reactants are comprised of vinyl chloride monomer hereinafter simply vinyl chloride, carbon monoxide, and an amidation agent, hereinafter termed amine. Vinyl chloride is possessed of the formula $H_2C{=}CHCl$. Vinyl chloride, also known as VCM, is widely commercially available.

Carbon monoxide employed in the practice of the invention desirably is free from other combustion products and particularly should be free of oxidizing agents. The absence of such other substances is desirable, if for no other reason, to provide a reduced interference to the carbon monoxide being combined with the other reactants and to improve the quantity of carbon monoxide that can be made available within the confines of a specific reactor volume.

The amidation agent or amine is selected from a group consisting of ammonia, primary amines and secondary amines. The primary and secondary

amines include one or more carbon chains each having at least one and not more than about ten carbon atoms. The carbon chain may be branched, unbranched, or cyclic, and the carbon chain may be saturated or unsaturated; it may be preferable that the carbon chain be saturated as under some conditions unsaturation may contribute to interfering reactions that could lower the effective yield of acylamide resulting from the practice of the process of the invention.

The catalyst preferred in the practice of the instant invention is a complex of a platinum group metal with phosphine, generally a teritiary phosphine, and typically possesses the formula: $Y_zM(L)_n$. M is preferably a platinum group metal, but may be cobalt and, with suitable ligands, it is believed iron or nickel. The platinum group comprises palladium, platinum, iridium, rhodium, ruthenium and osmium. L is of the form $PR_3$ or $[R_2P\text{-}A\text{-}PR_2]$. R is preferably aryl, substituted aryl, or alkyl such as straight or branched chain alkyl, cyclohexyl, teritary alkyl and derivatives thereof having a substituent X. R is typically a phenyl or cyclohexyl, t-butyl, or other alkyl of between 4 and 10 carbon atoms while X is selected from a group including methyl, fluoromethyl, lower molecular weight halogen, and hydrogen. R may include heteroatoms such as N, S, and O. In the practice of the invention it is much preferred that R be phenyl. A is a connecting organic radical, aliphatic or aromatic, of between 1 and 6 carbon atoms.

Integers, n can be 1-4 and z can be 0-2 in the practice of the invention. It is much preferred that z equal a number corresponding to the platinum group metal being in one of the following states: Pd(O), Pt(O), Rh(I), Ir(I), Ru(II) and Os(II) termed here catalytic states.

Where L is $PR_3$, n=2-4 and z=0-2. Where L is $[R_2P-A-PR_2]$, n=1-2 and z=0-2. Y typically is a monovalent anion such as a lower molecular weight halogen like chlorine, but may be divalent. Complex compounds fitting this $Y_zM(L)_3$ stoichiometry can be prepared directly or may generated in-situ from suitable metal precursors and the appropriate stoichiometric amount of phosphine.

Therefore, where n is less than a number required to place the platinum group metal in the catalytic state, it is much preferred in the practice of the invention that a stoichiometric quantity of the phosphine that is complexed with the platinum group metal be added into the combination of reactants to provide a phosphine presence in the combination at least equivalent to the presence that would have been achieved had sufficient phosphine been included in the platinum group metal complex to place the platinum group metal complex in the catalytic state. Particularly, where the amine is ammonia or a primary amine, the addition of this stoichiometric quantity of phosphine may provide a desirable effect upon the rate at which the reaction proceeds.

In situ generation of $Pd(PR_3)_n$ where n=2-4 can be accomplished from $Pd_3(TBAA)_3CHCl_3$ where TBAA is tribenzylidene acetylacetone and a stiochiometrically appropriate quantity of phosphine as shown by Ishi, Y., et al, 73 Journal of Organometallic Chemistry, pp 411 (1974) and 16-A Chubic Kogyo Kiyo, A, pp 103 (1980). In situ generation of $Pd(PR_3)_4$ where R is phenyl can be accomplished by amine reduction of $PdCl_2(PR_3)_2$ in the presence of $PR_3$ where the amine is either primary or secondary and includes at least one alkyl substituent.

Modest improvements in catalytic performance as evidenced by reaction rate are available particularly where R is phenyl and X is a strongly electron donating para substituent. Typically the reaction rate increases only by a factor 3-4 as X varies from strongly electron withdrawing to strongly electron donating. Where R is a bulky alkyl, such as t-butyl, significant rate increases may be observed.

The platinum group metal is selected from a group consisting of palladium, platinum, osmium rhodium, iridium, and ruthenium. Platinum, rhodium and palladium are preferred in the practice of the invention and palladium is much preferred in the practice of the invention.

The combination of reactants and catalyst must be heated to initiate a commercially reasonable rate of reaction to produce acrylamides. It is believed that for commercial utility the temperature must be elevated to at least 80°C and much preferably to at least 90°C to cause production of acrylamides at a significant rate. In the lab, meaningful reaction rates more typically require a temperature of at least 80°C. Maintaining the combination of reactants and catalysts at approximately 90-110°C during reaction has been found to produce satisfactory results. Reactor cooling may be required. Upper limitations on temperature, it is believed, are established by catalyst instability and the possibility for polymerization of the acrylamide being produced and/or the vinyl chloride monomer. Additionally, as the temperature of the combination of reactants and catalysts is elevated, the pressure associated with the combination becomes more elevated, and pressure limitations inherent to physical

limitations associated with the equipment in which the combination of reactants is being processed may establish an ultimate temperature ceiling. Additionally, a CO partial pressure in excess of about 2440 kPa (measured at 22°C) can cause a reduction in catalytic rate.

It is preferred that the combination of reactants be agitated during periods where reaction is desired thereby assisting in maintaining equilibrium during reaction, particularly where a liquid phase reaction is contemplated. It is also believed advantageous, particularly where ammonia is being employed as an amine in a liquid phase reaction, to agitate the vapor spaces of any reaction vessel in addition to the liquid phase to rapidly achieve vapor-liquid equilibrium. Agitation can be accomplished in any suitable or conventional well-known manner.

The principal product of the reaction is an acrylamide. It is believed that the reaction proceeds as follows:

$$CH_2{=}CHCl + 2HNR^{I}R^{II} + CO \longrightarrow$$

$$CH_2{=}CHCONR^{I}R^{II} + Cl^{-}H\overset{+}{N}R^{I}R^{II}$$

where $R^{I}$ and $R^{II}$ are either hydrogen, or branched, unbranched, or cyclic, saturated or unsaturated hydrocarbon chains having between one and about 10 carbon atoms. $R^{I}$ and $R^{II}$ need not be identical. Where the reaction is run in a low concentration of amines such as ammonia, primarily the acrylamide results from the catalytic reaction.

However, where a more elevated level of amine is present, a significant proportion of the

acrylamide produced by the catalytic reaction then further reacts with the amine to form a Michael addition product by the reaction

$$H_2C{=}CHCONR^IR^{II} + HNR^IR^{II} \longrightarrow R^IR^{II}NCH_2CH_2CONR^IR^{II}.$$

For example, where the amine is dimethylamine, the Michael addition product or adduct is 3-dimethylamino-N,N-dimethylpropanamide, but for ammonia 3,3',3''-nitrilotripropanamide is the addition product with acrylamide. The hydrochloride of the Michael adduct is also obtained. The Michael reaction may be reversed to obtain an acrylamide, accomplished according to well known techniques such as by heating under vacuum the Michael adduct to a temperature of about between 160°C and 200°C.

Catalyst is deactivated slowly during the reaction by a competing addition reaction with the acrylamide by which the phosphine ligand is consumed.

$$PR_3 + CH_2 = CHCONR^IR^{II} \xrightarrow{Cl^-H_2\overset{+}{N}R^IR^{II}}$$

$$Cl^-R_3\overset{+}{P}CH_2CH_2CONR^IR^{II} + HNR^IR^{II}$$

Palladium freed of phosphine may react with vinyl chloride in the combination of reactants but it is believed to separate from solution as metallic palladium and ceases to function catalytically. The reaction of the acrylamide with the phosphine appears to be somewhat dependent upon the concentration of acrylamide present in the combination of reactants. Therefore, any chemical or mechanical method for rapidly and effectively removing the acrylamide being formed from the reaction medium can improve catalyst stability.

Operating with an excess of an amine that adds to acrylamide very rapidly and/or the use of an elevated concentration of amine consistant with an acceptable rate of reaction can remove the acrylamide from the combination of reactants as a Michael reaction product and can thereby effectively reduce the drain posed by the competing reaction between phosphine and acrylamide upon the catalyst function. Excess phosphine, while depressing catalytic rates, can prolong catalyst life.

The form in which the catalyst is introduced into the combination of reactants can be important to the success of the reaction. Typically, the platinum group metal is in a state, or catalytic state as used herein of Pd(O), Pt(O), Rh(I), Ir(I), Ru(II) and Os(II). Where palladium is of the form $Pd(PR_3)_n$ (n=2-4). Typically for palladium, n = 4, but upon introduction of the palladium catalyst into vinyl chloride monomer, such palladium complexes are known to disassociate readily to yield coordinatively unsaturated complexes, the dissociation being at least in part a function of the phosphine ligands as set forth by Tolman, C.A., 77 Chem. Revs. pp 313 (1977). Such coordinatively unsaturated complexes are believed responsible for catalytic activity and, at least with vinyl chloride, the reaction is believed to occur by an oxidative addition process as follows:

1.) $Pd(PR_3)_4 \rightleftharpoons Pd(PR_3)_n + (4-n)PR_3$

2.) $Pd(PR_3)_n + H_2C{=}CHCl \longrightarrow H_2C{=}CH$

```
                                      \
                                       Pd(PR3)n
                                      /
                                     Cl
```

3.) $H_2C{=}CH$

$\quad\quad\backslash$

$\quad\quad Pd(PR_3)_n + CO + 2HNR^{I}R^{II} \longrightarrow$

$\quad\quad /$

$\quad\quad Cl$

$$H_2C{=}CHCONR^{I}R^{II} + Cl^{-}H_2\overset{+}{N}R^{I}R^{II} + Pd(PR_3)_n$$

It has been found that the rate at which the reactants form acrylamide is relatively insensitive to the basicity of the amine. It has been noticed that a greater initial concentration of ammonia, where employed as the amine in the reactant combination, can suppress the rate at which the reactants form acrylamide. This phenomenon is believed related to ammonia functioning as ligands for palladium thereby reducing the concentration of active, coordinatively unsaturated catalyst. Conversely, where dimethylamine is employed, a greater initial concentration of dimethylamine in the combination of reactants can result in a more rapid rate of reaction. It has been found that excessive quantities of carbon monoxide present in the combination of reactants can reduce the overall rate of reaction, it is believed, for the same reasons set forth for ammonia.

The reaction of the instant invention may be practiced in an apparatus as depicted in Figure 1. Referring to Figure 1, a reactor 10 (available from Autoclave Engineers as a so-called Zipperclave) made of stainless steel having a capacity of 500 milliliters is provided with a glass liner 12. The reactor 10 is provided with a rupture disk 14. A thermostated mantle 16 is provided, capable of receiving the 500 milliliter stainless steel reactor 10 for accurate control of temperature within the reactor 10. An air motor 17

driven agitator 18 is provided by which paddles 20 within the reactor may be activated.

A vacuum line 22 is provided upon the reactor including a metering cock 24, and a stop cock 26. A sampling line 28 including block valves 29 is provided. An inlet line 30 is provided including a U-tube 32, a pressure gauge 34, a three-way cock 36, including a port 37 for introducing gases, a metering valve 38, a carbon monoxide inlet cock 40 and block valves, 41, 42.

The reactor 10 and associated equipment were employed in the generation of acrylamides as set forth in the examples that follow.

Example 1. A 100 milliliter 3-neck flask was fitted with an inert gas inlet, a thermometer, a gas bubbler, a magnetic stirring bar, and a port including a rubber septum. The flask was purged with nitrogen and charged with 60 milliliters of DMSO, 6.55 grams (0.025 moles) of triphenylphosphine, and 0.885 grams (5.00 millimoles) of palladium chloride. Under agitation and an inerted atmosphere, here argon, the mixture was heated to 140°C to produce a solution. The mixture was then allowed to cool slowly under agitation. At 130°C 1.18 grams (0.020 moles) of 85% hydrazine hydrate was added employing a syringe over approximately two minutes. Allowed to crystalize, the product was filtered through a course scintered glass disk under nitrogen and was washed twice with 10 milliliter aliquots of ethanol followed by pentane washes. Drying was accomplished employing flowing nitrogen at room temperature to yield 5.43 grams (4.70 millimoles) of yellow crystals at a 94% yield. The resulting tetrakis(triphenylphosphine)palladium was employed in the practice of the invention.

Example 2. In a typical acrylamide reaction, the reactor 10 having a glass liner 12 was charged 0.36 moles tetrakis(triphenylphosphine)palladium catalyst and evacuated to less than 26 Pa employing the line 22. The reactor 10 was immersed in an ice bath; 63.4 grams (1.01 moles) vinyl chloride was condensed into the reactor by vapor transfer. The catalyst-VCM mixture was agitated while venting 800 ml of VCM vapor employing the line 28 as a purge leaving 61.4 grams (0.983 moles) of VCM in the reactor. The reactor was placed in the mantle 16. The U-tube 32 was evacuated, cooled in liquid nitrogen, and the three way cock 36 was opened to condense the amine, here ammonia, into the U-tube. The three way valve 36 was adjusted to permit flow from the U-tube 32 to the valve 42, and as the U-tube was warmed with hot tap water, the valve 42 was opened to admit the ammonia into the reactor 10. The reactor 10 was then rapidly pressurized with carbon monoxide with the agitator 18 idle through the valves 40, 41, 36, 42 and the U-tube 32. The partial pressure of carbon monoxide as noted herein is the difference between pressure in the reactor 10 immediately before and after introduction of carbon monoxide and before any heating or agitation. The reactor was heated to 100°C while stirred. Based upon known vapor pressure and density data, it was believed that about 53 milliliters of VCM remained in the liquid state after heating. Ammonia disappearance was monitered by gas chromotography employing a 0.32 cm x 183 cm silanized column packed with 80/100 mesh Poropak PS operated at 60°C. VCM was the internal standard. The sample was the piping volume between the valves 29 in the line 28 which was maintained at 120°C. At the conclusion of the reaction, the

mantle was removed and the reactor 10 was cooled in ice water. After venting (while stirred) and opening the reactor, the contents thereof were extracted with 50-100 milliters of $D_2O$ and the resulting solution filtered. The weight of the combined extract was determined and teriary-butanol was added as an internal reference for quantitative $^1H$ NMR analysis. VCl conversions were determined by potentiometric titration for chloride using silver nitrate (aqueous) and a chloride specific electrode. Reported yields are based upon these chloride titrations.

This Example was repeated varying the quantities of ammonia, carbon monoxide, and catalyst included in the reactor. The results are set forth in Table I wherein R in the catalyst is a phenyl group. The initial rate of ammonia consumption is the value reported in Table I.

TABLE I. Influence of Reaction Variables on VCl Amidation

| $P_{(CO)}$ (psi) | $NH_3$ (mol) | T (°C) | RATE($NH_3$) (mol $min^{-1}$) x$10^4$ | Pd($PR_3$*)$_4$ (mmol) | YIELD (%)[a] d | e | f | g | g,% of[b] THEOR. MAX | COMBINED YIELD(%) | N.BAL[c] (%) | VCl CONVER. (mmol) | RECHARGE $NH_3$ | REACTION TIME (min) |
|---|---|---|---|---|---|---|---|---|---|---|---|---|---|---|
| 240 | .35 | 100 | 5.3 | .36 | 81 | 16 | .24 | .04 | 1.4 | 96 | 109 | 49.8 | NO | 316 |
| 240 | .36 | 100 | 4.7 | .36 | 70 | 17 | 2.2 | .04 | 1.4 | 88 | 105 | 49.5 | NO | 221 |
| 240 | .20 | 100 | 5.9 | .36 | 87 | 11 | 2.7 | .8 | 56. | 101 | 105 | 77.9 | NO | 464 |
| 240 | .16 | 100 | 10. | .36 | 77 | 15 | 2.0 | .6 | 40. | 94 | 86 | 75.1 | YES | 341 |
| 240 | .12 | 100 | 13. | .36 | - | - | - | - | - | - | - | 50.6 | NO | 110 |
| 240 | .12 | 100 | 15. | .36 | - | - | - | - | - | - | - | - | NO | - |
| 240 | .074 | 100 | 16. | .36 | 0 | 16 | 66 | 5.7 | 100. | 88 | 80 | 27.9 | NO | 42 |
| 240 | .12 | 100 | 14. | .36 | with .034 mol d added | | | | - | - | - | - | NO | 85 |
| 320 | .12 | 100 | 6.8 | .36 | 79 | 13 | 5.3 | .9 | 49. | 102 | 93 | 81.4 | YES | 396 |
| 160 | .12 | 100 | 18. | .36 | 72 | 20 | 1.4 | 1.2 | 84. | 95 | 106 | 124. | YES | 433 |
| 120 | .13 | 100 | 18. | .36 | 40 | 10 | 31. | 3.2 | 100. | 85 | 84 | 53.4 | NO | 70 |
| 80 | .15 | 100 | 12. | .36 | 61 | 8 | 17. | 2.1 | 65. | 89 | 89 | 44.5 | NO | 102 |
| 160 | .12 | 90 | 4.1 | .36 | 71 | 10 | 3.3 | .7 | 28. | 86 | 86 | 56.8 | YES | 598 |
| 160 | .12 | 110 | 25. | .36 | 55 | 33 | .8 | 1.3 | 51. | 90 | 77 | 55.1 | YES | 211 |
| 160 | .18 | 100 | 12. | .36 | 77 | 17 | 2.2 | .7 | 34. | 97 | 85 | 70.9 | YES(f=.5) | 294 |
| 160 | .18 | 100 | 13. | .36 | 66 | 14 | 1.6 | 1.1 | 60. | 83 | 84 | 76.9 | YES(f=0) | 265 |
| 160 | .18 | 100 | 39. | .72 | 64 | 26 | nil | 1.2 | 62. | 92 | 84 | 150. | YES | 650 |

a. Based on VCl converted

b. Theoretical maximum is $PPh_3$ available from $Pd(PPh_3)_4$

c. Nitrogen in products ÷ ammonia consumption (gc)

d. $N(CH_2CH_2CONH_2)_3$

e. $Cl^- H\overset{+}{N}(CH_2CH_2CONH_2)_3$

f. acrylamide

g. $Cl^- R_3\overset{+}{P}CH_2CH_2CONH_2$

* R is phenyl

Example 3. Example 2 was repeated employing para-substituted phenyl groups in lieu of unsubstituted phenyl in the triphenylphosphine ligand of the catalyst. The results are set forth in Table II.

# 0185350



TABLE II. Para-Substituent Effect on Amidation

| P (CO) (psi) | $NH_3$ (mol) | T (°C) | Rate($NH_3$) (mol $min^{-1}$) $x10^{4}$ | R for $Pd(PR_3)_n$ R | $n^g$ | (mmol) | YIELD (%) a | b | c | d | d,% of Theor. max | Total Yield (%) | N Bal. (%) | VCl Conver. (mmol) | Recharge Ammonia | React. Time (min) |
|---|---|---|---|---|---|---|---|---|---|---|---|---|---|---|---|---|
| 240 | .12 | 100 | 7.6 | para-$C_6H_4CF_3$ | 4 | .36 | 51 | 12 | 3. | $.6^f$ | $7^f$ | 67 | 59 | 15.7 | no | 208 |
| 240 | .12 | 100 | 8.6 | para-$C_6H_4F$ | 4 | .36 | 72 | 9.3 | 0 | 0 | 0 | 81 | 54 | 36.5 | yes | 275 |
| 240 | .12 | 100 | $13.^e$ | $C_6H_5$ | 4 | .36 | 82 | 7.3 | 1.6 | 1.0 | 47 | 92 | 81 | 64.5 | yes | 350 |
| 240 | .12 | 100 | 27. | para-$C_6H_4CH_3$ | 4 | .36 | 63 | 22. | 0 | 2.9 | 90 | 88 | 66 | 45.2 | yes | 95 |

a. $N(CH_2CH_2CONH_2)_3$

b. $Cl^- H\overset{+}{N}(CH_2CH_2CONH_2)_3$

c. acrylamide

d. $Cl^-(X-$ 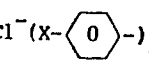 $-)_3\overset{+}{P}CH_2CH_2CONH_2$

e. Average of three runs.

f. Upper limit.

g. From $Pd(PR_3)_3$ and one equivalent of $PR_3$

Example 4. Example 2 was repeated employing alternate catalyst ligands wherein $Pd(PR_3)_n$ was generated in situ from $Pd_3(TBAA)_3CHCl_3$ with a variety of phosphorous containing ligands, that is $PR_3$, and a tertiary-stibene added in the stoichiometry indicated. The results are set forth in Table III wherein Ph represents phenyl.

TABLE III. Amidation Catalyzed by PdLn Generated in-situ From $Pd_3(TBAA)_3(CHCl_3)$ and L

| P(CO) (psi) | $NH_3$ (mol) | T (°C) | Rate (mol $min^{-1}$) x$10^4$ | Pd$(PR_3)_n$ $PR_3$ | n | (mmol) | Yield (%)[a] a | b | c | d | d,% of Theor. max | Total Yield (%) | N Bal. (%) | VCl Conver. (mmol) | Recharge Ammonia | React. Time (min) |
|---|---|---|---|---|---|---|---|---|---|---|---|---|---|---|---|---|
| 240 | .12 | 100 | 14 | $PPh_3$ [f] | 4 | .36 | 68 | 13 | 2.8 | .9 | 35 | 85 | 70 | 57.2 | yes | 180 |
| 240 | .12 | 100 | 0.1 | P(o-tolyl)$_3$ | 2 | .36 | - | - | - | - | - | - | - | .8 | no | 134 |
| 240 | .12 | 100 | 0.05 | $Ph_2PCH_2CH_2PPh_2$ | 2 | .36 | - | - | - | - | - | - | - | .4 | no | 171 |
| 240 | .12 | 100 | 0.05 | ortho-$(PPh_2)_2C_6H_4$ | 2 | .36 | - | - | - | - | - | - | - | .5 | no | 257 |
| 240 | .12 | 100 | 37[e] | P(cyclohexyl)$_3$ | 4 | .36 | 14 | 3.2 | 63. | - | - | 80 | 76 | 50. | no | 190 |
| 240 | .12 | 100 | 71 | P(cyclohexyl)$_3$ | 2 | .36 | 63 | 12. | 3. | - | - | 69 | 69 | 92.9 | yes | 228 |
| 240 | .12 | 80 | 8.8 | P(cyclohexyl)$_3$ | 2 | .36 | - | - | - | - | - | - | - | 57.5 | yes | 305 |
| 240 | .12 | 100 | 0 | Sb(p-toluene)$_3$ [g] | 4 | .36 | - | - | - | - | - | - | - | 0 | no | 200 |
| 240 | .12 | 100 | 0.02 | P(OPh)$_3$ | 2 | .36 | - | - | - | - | - | - | - | 1.8 | no | 225 |
| 240 | .12 | 100 | 28 | $PPh_3$ | 2 | .36 | - | - | - | - | - | - | - | - | - | - |
| 240 | .12 | 100 | ·0.1 | P(1-napthyl)$_3$ | 4 | .36 | - | - | - | - | - | - | - | 1.1 | no | 250 |
| 240 | .12 | 90 | 43 | P(tertiary-butyl)$_3$ | 4 | .36 | 31 | 4.4 | 48. | 4.7 | 100 | - | - | 38.2 | no | 126 |

a. $N(CH_2CH_2CONH_2)_3$

b. $Cl^-\overset{+}{N}H(CH_2CH_2CONH_2)_3$

c. acrylamide

d. $Cl^-(R_3)\overset{+}{P}CH_2CH_2CONH_2$

e. 170 min induction period followed by fast reaction

f. Ph is phenyl

g. Sb substitutes for P

Example 5. Example 2 was repeated employing aniline and dimethylamine in lieu of ammonia. The results are set forth in Table IV.

TABLE IV. VCl Amidation with Dimethylamine and Aniline

| P (CO) | Amine (mol) | T(°C) | Rate (mol $min^{-1}$) $x10^4$ | $Pd(PPh_3)_4$ (mmol) | Yield (%) d | Yield (%) d·HCl | Yield (%) e | Yield (%) f | VCl Conver. (mmol) | Recharge Amine | React. Time (min) |
|---|---|---|---|---|---|---|---|---|---|---|---|
| 240 | Dimethylamine (.12) | 100 | 39 | .36 | - | 94 | - | 0 | 158 | yes | 44 |
| 240 | Dimethylamine (.12) | 90 | 14 | .36 | 100[a] | - | - | 0 | 41.6 | no | 86 |
| 240 | Dimethylamine (.12) | 90 | 14 | .36 | - | - | - | - | - | - | - |
| 240 | Dimethylamine (.059) | 90 | 7.6 | .36 | - | - | - | - | - | - | - |
| 240 | Dimethylamine (.059) | 90 | 9.5 | .36 | - | - | - | - | - | - | - |
| 240 | Dimethylamine (.24) | 90 | 24.[b] | .36 | - | - | - | - | - | - | - |
| 240 | Dimethylamine (.12) | 100 | 15. | .18 | - | - | - | - | - | - | - |
| 240 | Dimethylamine (.12) | 90 | 6.0 | .087 | 100 | - | - | $\leq$.2 | 33 | - | 178 |
| 240 | Dimethylamine (.12) | 90 | 11.0 | .18 | - | - | - | - | - | - | - |
| 240 | Dimethylamine (.12) | 90 | 5.6 | .70 | - | - | - | - | - | - | - |
| 240 | Dimethylamine (.12) | 90 | 17.0 | 1.1 | - | - | - | - | - | - | - |
| 240 | Dimethylamine (.12) | 70 | 0.72 | 0.71 | - | - | - | - | .24 | no | 1250 |
| 240 | Aniline (.12) | 100 | 2.8[c] | .36 | - | - | - | - | 9.75 | no | 105 |
| 240 | Aniline (.12) | 100 | 3.3[c] | .36 | - | - | 81 | - | 37.7 | no | 320 |
| 240 | Aniline (.12) | 100 | 3.6[c] | .36 | - | - | - | - | 41.8 | no | 376 |

a. Mixture of 5d and 5d·HCl

b. Approx. 30 min induction period

c. Estimated from VCl conversion with stoichiometry, aniline/VCl consumption = 3.

d. $R^IR^{II}NCH_2CH_2CONR^IR^{II}$, $R^I=R^{II}=CH_3$

e. $R^IR^{II}NCH_2CH_2CONR^IR^{II}$, $R^I=H$, $R^{II}=C_6H_5$

f. $Cl^-(PPh_3)_3\overset{+}{P}CH_2CH_2COR^IR^{II}$

Ph = phenyl

Example 6. Example 2 was repeated employing alternate catalyst systems including platinum group metals and cobalt. The results are summarized in Table V.

TABLE V. Activity of Platinum Group and Related Metal Complexes in VCl Amidation[a]

| Catalyst (mmol) | P(CO) (psi) | $NH_3$ (mol) | VCl Converted (mmol) | React. Time (HR) | Turnover[b] Rate ($HR^{-1}$) |
|---|---|---|---|---|---|
| $Pd(PPh_3)_4$ (.36) | 240 | .12 | 50.6[c] | 1.51 | 93. |
| $Pt(PPh_3)_4$ (.36) | 240 | .12 | 1.4 | 3.25 | 1.2 |
| $Pt(PPh_3)_3$ (.36) | 240 | .12 | 1.7 | 1.67 | 2.8 |
| $Ir(PPh_3)_2(CO)Cl$ (.36) | 240 | .12 | 2.6 | 3.17 | 2.3 |
| $Rh(PPh_3)_3H(CO)$ (.36) | 240 | .12 | 1.8 | 2.5 | 2.0 |
| $Ru(PPh_3)_3Cl_2$ (.36) | 240 | .12 | 0.5 | 3.85 | 0.5 |
| $Co(PPh_3)_3Cl$ | 240 | .12 | 0.74 | 3.45 | 0.6 |

a. At 100°C.

b. mol VCl converted·mol $cat^{-1}\cdot hr^{-1}$.

c. Calculated from rate of ammonia consumption.

Ph = phenyl

Example 7. Example 2 was repeated employing a catalyst of the form $PdCl_2(PPh_3)_2$; in each repetition .36 millimoles of catalyst and .12 moles of ammonia were introduced into the reactor and the reactor was pressurized to a carbon monoxide partial pressure of 240 pounds per square inch and the reaction was maintained at 100°C for approximately 280 minutes. The results compared with results for $Pd(PPh_3)_4$ are as follows:

| Catalyst | Turnover Rate ($mol.VCM \cdot hr^{-1} \cdot mol.cat^{-1}$) |
|---|---|
| $Pd(PPh_3{}^*)_4$ | 93. |
| $PdCl_2(PPh_3)_2$ | $\leq .80$ |
| $PdCl_2(PPh_3)_2 + 2PPh_3$ | $\leq .50$ |

* Ph being phenyl

Example 8. The effect of methyl substitution on vinyl chloride was determined by repeating Example 2 with the vinyl chloride being replaced in three separate examples by 48 grams of cis-, trans-, and 2-chlorpropene respectively, the quantities being selected to give approximately the same liquid volume in the reactor as obtained with the vinyl chloride monomer at 100°C. Reactions utilizing chloropropenes were conducted at 100°C for approximately 280 minutes and the results are compared with those for vinyl chloride as follows:

| Reactant | Rate[a] (relative) | Products | Yield % (mol basis) |
|---|---|---|---|
| $H_2C=CHCl$ | 74 | $N(CH_2CH_2CONH_2)_3$ | 71 |
| | | $Cl^- H\overset{+}{N}(CH_2CH_2CONH_2)_3$ | 7 |
| | | Acrylamide | 7 |
| | | $Cl^- Ph_3\overset{+}{P}CH_2CH_2CONH_2$* | 1 |
| cis-$(CH_3)HC=CHCl$ | 2. | cis-$(CH_3)HC=CHCONH_2$ | 66 |
| trans-$(CH_3)HC=CHCl$ | 1. | trans-$(CH_3)HC=CHCONH_2$ | 51 |
| $H_2C=C(CH_3)Cl$ | 1. | $H_2C=C(CH_3)CONH_2$ | 90 |

a This is a rate calculated from chloroalkene conversion by chloride titration.

* Ph is phenyl

Example 9. The effect of solvent upon the reaction of Example 2 was investigated by repeating Example 2 employing .12 moles of ammonia, .36 moles of $Pd(PPh_3)_4$ catalyst where Ph is phenyl and charging the reactor employing a 1655 kPa partial pressure carbon monoxide. In one such run, the 19.1 grams of vinyl chloride charged to the reactor was augmented with 26.1 grams of acetonitrile and in another run the 19.1 grams vinyl chloride charged was augmented by 30.7 grams of toluene. The solvent quantities were selected to produce approximately the same volume within the reactor at 100°C as is present when employing vinyl chloride. The reaction rates as measured by ammonia disappearance in $mol \cdot min^{-1} \cdot 10^4$ were 13.0, 6.8 and 10.0 for vinyl chloride, acetonitrile/vinyl chloride, and toluene/vinyl chloride respectively. The product mix from the acetonitrile/vinyl

chloride run yielded nitrilotripropanamide (57% on a mol basis), nitrilotripropanamide.HCl (18% on a mol basis), acrylamide (5.7% on a mol basis), and phosphonium salt (3.6 of on a mol basis).

Example 10. Example 2 was repeated employing as the amine .12 moles of dimethylamine, and as catalyst .36 millimoles of $Pd(Ph_2PCH_2CH_2PPh_2)$ generated in situ from 0.184 grams of $Pd_3(TBAA)_3CHCl_3$ and .148 grams of 1,2-bis(diphenylphosphino)ethane. Conversion of the dimethylamine was 86% by weight in 21 hours after which an additional 0.10 mole of dimethylamine was charged to the reactor together with sufficient additional carbon monoxide so that the original total pressure was restored to the reactor at 90°C. The reaction was stopped after a total reaction time of 26 hours. The initial rate of dimethylamine consumption as measured by gas chromotography through a 26% conversion was $1.1 \times 10^{-4}$ mol·min$^{-1}$. This rate compares with $14 \times 10^{-4}$ mol·min$^{-1}$ obtained under the same conditions using $Pd(PPh_3)_4$. The reaction product was 3-dimethylamino-N,N-dimethylpropanamide at a 98% yield by weight.

Example 11. Example 2 was repeated employing as the amine 21.9 grams (0.12 moles) of di-n-hexylamine the reaction being conducted for 20 hours. Following cooling and venting, the residue was stirred with 55 milliliters of ethanol and filtered. As measured by chloride titration, vinyl chloride conversion was 52 millimoles. Mass spectrometry showed the product to comprise di-n-hexylammonium chloride, N,N-di-n-hexylacrylamide,

3-(di-n-hexylamino)-N,N-di-n-hexylpropanamide and little or no unreacted di-n-hexylamine. A fraction of the ethanol extract was evaporated to dryness and dissolved in methanol-$d_4$. Toluene was added as an internal reference for quantitative $^1H$ NMR analysis which revealed a product mix of N,N-di-n-hexylacrylamide (79% on a mol basis) and 3-(di-n-hexylamine)-N,N-di-n-hexylpropanamide (29% on a mol basis), the latter being estimated from overlapping peaks. Thus even extended chain primary and secondary amines can be employed in the practice of the instant invention.

From the foregoing examples it should be apparent that large and small alkylamines and weakly basic aromatic amines find utility in the practice of the instant invention. Further, particularly with palladium based catalyst, the reaction proceeds quite rapidly and can provide an extended catalyst life. Particularly, some dialkylamines possess the capability for rapidly removing acrylamide by Michael addition and rapidly reducing $Pd^{2+}$ complexes to Pd(O) complexes under reaction conditions.

While a preferred embodiment of the invention has been shown and described in detail, it should be apparent that various modifications may be made thereto without departing from the scope of the claims that follow.

## CLAIMS

What is claimed is:

1. A process for making acrylamides comprising the steps of:

combining as reactants: a) vinyl chloride monomer; b) carbon monoxide; c) an amine selected from a group consisting of: ammonia and primary and secondary amines having organic radicals of at least one and not more than about ten carbon atoms, the radicals being one of branched, unbranched, and cyclic and being one of saturated and unsaturated; and a catalyst of the form $Y_zM(L)_n$ wherein n=1-4, Y is an anion, z=0,1,2, M is one of a platinum group metal selected from a group consisting of palladium, platinum, iridium, rhodium, ruthenium, osmium and mixtures thereof and cobalt; and L is one of $PR_3$ and $[R_2P\text{-}A\text{-}PR_2]$ wherein R is one of alkyl, aromatic and substituted aromatic radicals and A is a connecting organic radical of 1 to 6 carbons;

maintaining the combination under agitation at a temperature at least in excess of about 60°C under a pressure; and

recovering the acrylamide from the combination.

2. The process of claim 1 including the steps of effecting a reversal of any Michael adduct formed as a product of the addition of acrylamide formed in the reaction with the amine by heating the Michael adduct to a temperature sufficient to effect the reversal to yield an acrylamide.

3. The process of either one of claims 1 and 2 including the step of replenishing the reactants and catalyst within the combination.

4. The process of claim 3, wherein R includes a substituent X, R being one of phenyl, alkyl and cyclohexyl and X being selected from a group consisting of hydrogen, methyl, a low molecular weight halogen and fluoromethyl.

5. A process for making acrylamides comprising the steps of:

combining as reactants: a) vinyl chloride monomer; b) carbon monoxide; and c) an amine selected from a group consisting of: ammonia and primary and secondary amines having organic radicals of at least one and not more than ten carbon atoms, the radical being one of branched, unbranched, and cyclic, and being one of saturated and unsaturated, together with a catalyst of the form $Y_zM(L)_n$ wherein n=1-4, M is a platinum group metal selected from a group consisting of palladium, platinum, rhodium, and mixtures thereof, L is one of $PR_3$ and $[R_2P\text{-}A\text{-}PR_2]$ with R including a substituent X, R being one of phenyl, alkyl and cyclohexyl and X being selected from a group consisting of hydrogen, methyl, fluorine, chlorine and fluoromethyl, z being 0,1,2; Y being an anion; and A being a connecting organic radical of 1-6 carbon atoms;

maintaining the combination under agitation under at least autogenous pressure at a temperature of at least about 60°C;

recovering the acrylamide and its derivatives from the combination; and

replenishing the reactants and catalyst within the combination.

6. The process of claim 5 including the step of thermally converting derivative amine-acrylamide adducts formed by the process, to acrylamide.

7. The process of claim 5, the amine being present in the combination in a mole ratio of not less than 0.02 relative to the vinyl chloride,

the CO being present at a partial pressure of at least 275 kPa, and

The catalyst being present in a quantity of not less than 0.10 millimoles per mole of liquid vinyl chloride.

8. The process of any one of claims 5, 6 and 7, the amine being one of $NH_3$ and dimethylamine.

9. A reaction product of: a) vinyl chloride monomer; b) carbon monoxide; and c) an amine selected from a group consisting of ammonia and primary and secondary amines, the primary and secondary amines having organic radicals, the radicals being one of unbranched, cyclic and branched and one of saturated and unsaturated, and such organic radical having at least one and not more than about 10 carbon atoms; reaction being conducted in the presence of a catalyst of the form $Y_zM(L)_n$; M being a platinum group metal selected from a group consisting of palladium, platinum, iridium, rhodium, ruthenium, osmium, and mixtures thereof; L being of the form $PR_3$ or $[R_2P\text{-}A\text{-}PR_2]$; R having a substituent X wherein R is one of phenyl, alkyl and cyclohexyl and X is selected from a group consisting of hydrogen, methyl, chlorine, fluorine, and fluoromethyl; n is not less than 1 nor more than 4; z=0,1,2; Y is a monovalent anion; A is one of an aliphatic and aromatic connecting organic radical of 1-6 carbon atoms.

10. The product of claim 9, the amine being one of dimethylamine and ammonia.

11. The product of any one of claims 9-10, M being palladium, R being phenyl and n being 4.

12. The method of any one of claims 1-2, and 5-7, the reaction being conducted in a homogenous liquid phase.

0185350

1/1

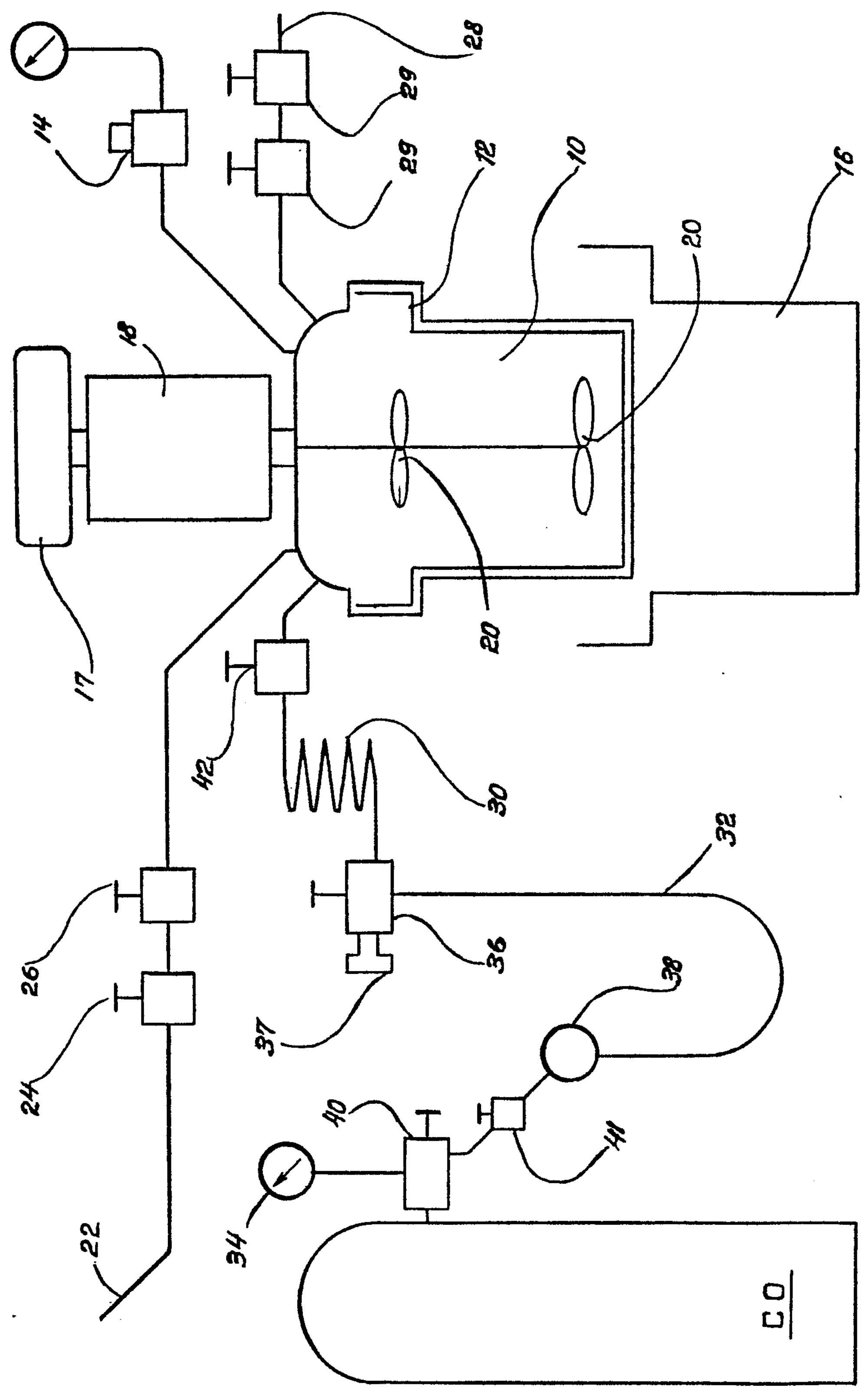


FIG. 1